# EUROPÄISCHE PATENTANMELDUNG

(11) **EP 1 857 449 A1**
(43) Veröffentlichungstag der Anmeldung: **21.11.2007**
(21) Anmeldenummer: 07108005.5
(22) Anmeldetag: 11.05.2007
(51) Int. Cl.: C07D 307/60

(54) **VERFAHREN ZUR HERSTELLUNG VON MALEINSÄUREANHYDRID IN EINEM MIKROKANAL-REAKTOR**

(30) Priorität: 18.05.2006 EP 06114165
(71) Anmelder: BASF Aktiengesellschaft, 67056 Ludwigshafen (DE)
(72) Erfinder: Wilmer, Hagen, 67071, Ludwigshafen (DE); Mäurer, Torsten, 67245, Lambsheim (DE); Rosowski, Frank, 68239, Mannheim (DE); Schuch, Rudolf, 69198, Schriesheim (DE)

(57) **Zusammenfassung**

Die vorliegende Erfindung betrifft ein Verfahren zur Herstellung von Maleinsäureanhydrid durch heterogenkatalytische Gasphasenoxidation eines Kohlenwasserstoffs, das dadurch gekennzeichnet ist, dass man einen Kohlenwasserstoff enthaltenden Stoffstrom und einen Sauerstoff oder eine Sauerstoffquelle enthaltenden Stoffstrom einem Mikrokanal-Reaktor zuleitet und in dem den Katalysator enthaltenden Mikrokanal-Reaktor die Umsetzung zu Maleinsäureanhydrid im Explosionsbereich erfolgt.

## Beschreibung

Die vorliegende Erfindung betrifft ein Verfahren zur Herstellung von Maleinsäureanhydrid durch heterogenkatalytische Gasphasenoxidation eines Kohlenwasserstoffs, das dadurch gekennzeichnet ist, dass man einen Kohlenwasserstoff enthaltenden Stoffstrom und einen Sauerstoff oder eine Sauerstoffquelle enthaltenden Stoffstrom einem Mikrokanal-Reaktor zuleitet und in dem den Katalysator enthaltenden Mikrokanal-Reaktor die Umsetzung zu Maleinsäureanhydrid im Explosionsbereich erfolgt.

Maleinsäureanhydrid ist ein wichtiges Zwischenprodukt bei der Synthese von γ-Butyrolacton, Tetrahydrofuran und 1,4-Butandiol, welche ihrerseits als Lösungsmittel eingesetzt werden oder beispielsweise zu Polymeren, wie Polytetrahydrofuran oder Polyvinylpyrrolidon weiterverarbeitet werden.

Die Herstellung von Maleinsäureanhydrid durch Oxidation von Kohlenwasserstoffen wie n-Butan, n-Butenen, Benzol oder Propan an geeigneten Katalysatoren ist seit langem bekannt. Im Allgemeinen werden hierzu Vanadium-, Phosphor- und Sauerstoff enthaltende Katalysatoren (siehe Ullmann's Encyclopedia of Industrial Chemistry, 6th edition, 2000 electronic release, Chapter "MALEIC AND FUMARIC ACIDS, Maleic Anhydride - Production"), Vanadium- und Molybdän-Mischoxide enthaltende Katalysatoren (Tang et al., Appl. Catal. A 287 2005 197), oder Molybdän- und Vanadium-Mischoxiden enthaltende Katalysatoren (Nikolov V., Hungarian Journal of Industrial Chemistry (2000), 28(4); Bordes E., Topics in Catalysis (2000), 11/12(1-4); Zazhigalov V. A., Theoretical an Experimental Chemistry (Translation of Teoreticheskaya i Eksperimental'naya Khimiya) (2000) Volume Date 1999, 35(5); Guliants Vadim V., Catalysis Today (1999), 51 (2); Cavani F., Trifiro F., Studies in Surface Science and Catalysis (1997), 110 (3rd World Congress on Oxidation Catalysis, 1997), 19-34; Cavani F., Cortelli C., Ligi S., Pierelli F.; Trifido F., DGMK Tagungsbericht -/3 87-100 (2004); Guliants Vadim V., Carreon Moises A., Catalysis (2005), 18, 1-45) eingesetzt.

Da die Oxidation der genannten Kohlenwasserstoffe zu Maleinsäureanhydrid stark exortherm ist, wird die Umsetzung in der Regel in einem salzbad-gekühlten Festbett-Rohrbündelreaktor durchgeführt. Je nach Größe der Anlage weist dieser wenige tausend bis mehrere zehntausend mit Katalysator gefüllte Rohre auf. Die gebildete Reaktionswärme wird über die Wandung der Katalysator-gefüllten Rohre an das umgebende Salzbad, in der Regel eine eutektische Mischung von Kalium- und Natriumnitrat und -nitrit, übertragen und abgeführt. Trotz dieser Salzbad-Kühlung stellt sich über die Länge der Katalysator-gefüllten Rohre keine einheitliche Temperatur ein. Es kommt zur Ausbildung überhitzter Bereiche, sogenannter Heißpunkte ("Hot Spots"). So ist die Kohlenwasserstoff-Konzentration des Reaktionsgemisches in der Nähe der Eintrittsstelle der Katalysator-gefüllten Rohre am höchsten und in der Nähe der Austrittsstelle am niedrigsten, was zur Bildung der genannten überhitzten Bereiche in der ersten Hälfte des Katalysatorbettes führt.

Ferner beeinflusst die übermäßige thermische Belastung die Katalysatorleistung und die Katalysatorlebensdauer. Da mit steigender Temperatur auch die Reaktionsgeschwindigkeit steigt und somit noch mehr Wärme produziert wird, kann die Bildung überhitzter Bereiche schließlich zu einem unkontrollierten Reaktionsverlauf führen, der ein "explosionsartiges Durchgehen" der Reaktion zur Folge haben kann.

Aus wirtschaftlichen und sicherheitstechnischen Gründen wird der Rohrbündelreaktor daher derart betrieben, dass die Reaktionstemperatur zur Sicherstellung einer wirtschaftlich attraktiven Ausbeute so hoch wie möglich ist, jedoch die in den einzelnen Rohren sich einstellenden überhitzten Bereiche nicht zu einem unkontrollierten Reaktionsverlauf führen.

Demnach ist die Herstellung von Maleinsäureanhydrid hinsichtlich der Prozessführung aus sicherheitstechnischen Gründen eingeschränkt. Es müssen beispielsweise bei der Wahl der Eduktzusammensetzung Explosionsgrenzen berücksichtigt und maximale Drücke eingehalten werden. Im Falle der Herstellung von Maleinsäureanhydrid im Festbett werden z. B. n-Butan-Konzentrationen auf maximal 1,5 bis 2,4 Vol.-% beschränkt und Drücke bis maximal 3 bis 5 bar angewendet. Der Einsatz konventioneller Reaktoren bei höheren Drücken ist nicht wirtschaftlich, weil die Investitionskosten aufgrund des Explosionsschutzes mit dem Druck stark zunehmen.

Ansatzpunkte zur Ausbeutesteigerung finden sich üblicherweise in der Optimierung der Prozessführung durch beispielsweise einer Aktivitätsstrukturierung des Katalysatorbetts mittels Geometrie und/oder Dotierung oder durch eine Temperaturstrukturierung des Katalysatorbettes oder durch Rezyklierung des Butans bzw. des Gemisches von Butan mit Luft und/oder Wasser und/oder durch Optimierung der verwendeten Katalysatoren, die speziell für den Einsatz im jeweiligen Reaktorkonzept abgestimmt werden. Die Optimierung der Katalysatoren erfolgt beispielsweise durch Zugabe von Dotiermetallen oder durch spezielle Herstellverfahren des Katalysators oder durch Zugabe von Porenbildnern.

In EP-A 593 646 wird ein Verfahren zur Herstellung von Maleinsäureanhydrid beschrieben, in dem die Katalysatoraktivität pro Volumeneinheit des Bettes mit der Temperatur und der Kohlenwasserstoffkonzentration in Fließrichtung des Gases variiert wird. Die Katalysatoraktivität wird derart eingestellt, dass die Reaktionsgeschwindigkeit durch eine hohe Aktivität in einem Bereich von niedriger Temperatur und geringer Kohlenwasserstoffkonzentration innerhalb des Bettes gefördert wird und durch eine relativ niedrige Aktivität in einem kritischen Bereich innerhalb des Bettes, wo die Kombination aus Temperatur und Wasserstoffkonzentration ansonsten ein Ablaufen der Umsetzung mit übermäßiger Geschwindigkeit oder ein übermäßiges Ansteigen der Gastemperatur hervorrufen könnte, beschränkt wird. In den Beispielen wird eine Ausbeute von 57 bis 59 % bei 2,0 mol-% Butan, einer GHSV von 1600 bis 1650 h⁻¹und einem Druck von 2 bis 2,14 bar erreicht.

In US 5,168,090 wird eine geformte Oxidationskatalysatorstruktur zur Herstellung von Maleinsäureanhydrid beschrieben, die aus gemischten Oxiden von Vanadium und Phosphor bestehendes katalytisches Material enthält und (i) ein geometrisches Volumen von 30 Prozent bis 67 Prozent von dem, das die hohlraumfreie massive geometrische Form aufweist, (ii) ein Verhältnis äußere geometrische Oberfläche/geometrisches Volumen von mindestens 20 cm⁻¹, (iii) eine Schüttdichte von 0,4 g/cm³ bis 1,4 g/cm³ und (iv) eine ausreichende mechanische Festigkeit aufweist. In den Beispielen wird bei 1,5 mol-% Butan, einem Druck von 1,034 barg, bei 2000 h⁻¹ GHSV, eine Ausbeute von 53 % bei einem Umsatz von 87 % erreicht.

In EP-A 876 212 wird ein Phosphor-Vanadium-Oxid-Katalysator zur Herstellung von Maleinsäureanhydrid beschrieben, wobei der Katalysator einen Formkörper mit einem Volumen von wenigstens 0,01 cm³ und einer BET-Oberfläche von wenigstens 15 m²/g umfasst und besagter Katalysator Molybdän enthält, und ein Molverhältnis von Molybdän zu Vanadium von zwischen 0,002 und 0,006 aufweist, wobei Molybdän im wesentlichen auf der Oberfläche des Katalysators konzentriert ist. In den Beispielen wird bei maximal 2,4 % Butan, einer Belastung von 2200 Nml pro Gramm Katalysator, 1,03 barg und 85 % Umsatz eine maximale Ausbeute von 58,6 mol-% erzielt.

In EP-A 1 219 352 wird ein Verfahren zur Herstellung eines Phosphor-Vanadiumoxid-Katalysators beschrieben, wobei zur Herstellung der Katalysatorvorstufe teilchenförmiges Porenmodifizierungsmittel in ausreichenden Verhältnissen verwendet wird, um eine Konzentration des Porenmodifizierungsmittels von 8 bis 16 Gew.-% zu schaffen, und wobei der Vorstufenkörper mit einer Geschwindigkeit zwischen 1°C und 3°C pro Minute auf eine Haltetemperatur erhitzt wird, die nicht mehr als 15°C unter einer Schwellentemperatur liegt. In den Beispielen wird bei 2,4 % Butan, einer GHSV von 1500/h, 1,034 barg und 85 % Umsatz eine maximale Ausbeute von 61 mol-% erzielt.

Ein weiterer Nachteil beim Einsatz von Rohrbündelreaktoren ist, dass aufgrund des uneinheitlichen Temperaturprofils die Präformierung der Katalysatoren nicht in dem Rohrbündelreaktor stattfinden kann, sondern typischerweise vor dem Füllen der Rohrbündelreaktoren in einem Ofen (EP-A 641 256) oder Bandkalzinierer (WO 03/78310) durchgeführt wird.

In US 2004/0220434 wird erstmalig der Einsatz von Mikrokanal-Reaktoren bei der Umsetzung von n-Butan zu Maleinsäureanhydrid beschrieben. Die Umsetzung erfolgt bei einem Eduktstrom aus n-Butan und Wasser in einem Volumenverhältnis von 1:1 und Luft, wobei die Luft mit den Reaktanden in einem Volumenverhältnis von 98:2 vermischt wird. In US 2004/0220434 wird folglich nicht im Explosionsbereich gearbeitet. Die Explosionsgrenze von n-Butan liegt bei 1,4 % bei 20 °C und 1 bar Luft.

In der Doktorarbeit von Herrn Kah mit dem Titel "Entwicklung und Einsatz von Mikrostrukturreaktoren mit katalytisch wirksamen Strömungskanälen für die partielle Gasphasen-Oxidation von 1-Buten" wird der Einsatz von Mikrokanal-Reaktoren für die Umsetzung von 1-Buten zu Maleinsäureanhydrid erforscht. Die vorgenommenen Reaktionen wurden bei 100 kPa Gesamtdruck und einer n-Buten-Konzentration von 0,45 und 5 Vol.-% in Luft bzw. 5 Vol.-% in Sauerstoff im Temperaturbereich von 355 bis 445 °C durchgeführt. In den Mikrostrukturreaktoren wurde eine maximale Selektivität zu Maleinsäureanhydrid von 33 % bei einem Umsatz von 83 % erzielt. Im Rahmen der gewählten Versuche konnte kein vorteilhaftes Verhalten der Reaktionsführung im Explosionsbereich gefunden werden (Explosionsgrenzen von 1-Buten in Luft nach GESTIS Datenbank: 1,2 (UEG) - 10,6 (OEG) Vol.-% in Luft).

Trotz des umfangreichen Stands der Technik auf dem Gebiet der Katalysatorforschung besteht ein ständiger Optimierungsbedarf in Hinblick auf eine verbesserte Ausbeute bei hoher Kapazität, insbesondere bei den aktuell steigenden Butanpreisen. Demnach bestand die Aufgabe der vorliegenden Erfindung darin, ein Verfahren zur Herstellung von Maleinsäureanhydrid aufzuzeigen, das im Vergleich zum Stand der Technik höhere Ausbeuten bei hoher Kapazität aufweist. Eine weitere Aufgabe bestand darin, einen Reaktor aufzuzeigen, in dem sowohl die Präformierung der Katalysatoren als auch die Umsetzung zu Maleinsäureanhydrid durchgeführt werden kann.

Es wurde gefunden, dass ein Verfahren zur Herstellung von Maleinsäureanhydrid durch heterogenkatalytische Gasphasenoxidation eines Kohlenwasserstoffs, das dadurch gekennzeichnet ist, dass man einen Kohlenwasserstoff enthaltenden Stoffstrom und einen Sauerstoff oder eine Sauerstoffquelle enthaltenden Stoffstrom einem Mikrokanal-Reaktor zuleitet und in dem den Katalysator enthaltenden Mikrokanal-Reaktor die Umsetzung zu Maleinsäureanhydrid im Explosionsbereich erfolgt, eine überdurchschnittliche Ausbeute an Maleinsäureanhydrid aufweist.

Als Kohlenwasserstoffe werden im erfindungsgemäßen Verfahren vorteilhaft aliphatische und aromatische, gesättigte und ungesättigte Kohlenwasserstoffe mit mindestens drei Kohlenstoffatomen eingesetzt, beispielsweise Propan, 1,3-Butadien, 1-Buten, 2-cis-Buten, 2-trans-Buten, n-Butan, C₄-Gemisch, 1,3-Pentadien, 1,4-Pentadien, 1-Penten, 2-cis-Penten, 2-trans-Penten, n-Pentan, Cyclopentadien, Dicyclopentadien, Cyclopenten, Cyclopentan, C₅-Gemisch, Hexene, Hexane, Cyclohexan und Benzol. Bevorzugt eingesetzt werden Propan, 1-Buten, 2-cis-Buten, 2-trans-Buten, n-Butan, Benzol oder deren Mischungen, insbesondere Propan, n-Butan oder Benzol. Besonders bevorzugt ist der Einsatz von n-Butan, beispielsweise als reines n-Butan oder als Komponente in n-Butan-haltigen Gasen und Flüssigkeiten. Das verwendete n-Butan kann beispielsweise aus dem Erdgas, aus Steamcrackern oder FCC-Crackern stammen.

Die Zugabe des Kohlenwasserstoffs erfolgt im allgemeinen mengengeregelt, d.h. unter stetiger Vorgabe einer definierten Menge pro Zeiteinheit. Der Kohlenwasserstoff kann in flüssiger oder gasförmiger Form dosiert werden. Bevorzugt ist die Dosierung in flüssiger Form mit anschließender Verdampfung vor Eintritt in die Rohrbündelreaktor-Einheit.

Als Oxidationsmittel werden Sauerstoff enthaltende Gase, wie beispielsweise Luft, synthetische Luft, ein mit Sauerstoff angereichertes Gas oder auch sogenannter "reiner", z. B. aus der Luftzerlegung stammender Sauerstoff eingesetzt. Auch das Sauerstoff-enthaltende Gas wird mengengeregelt zugegeben.

Das erfindungsgemäße Verfahren wird bei einer Temperatur von 250 bis 500 °C durchgeführt. Unter der genannten Temperatur ist, unabhängig von der Art des Reaktors, jeweils die mittlere Temperatur des Wärmeträgermediums zu verstehen. Im Falle der Verwendung von n-Butan als Kohlenwasserstoff-Edukt wird das erfindungsgemäße Verfahren bevorzugt bei einer Temperatur von 380 bis 460 °C und besonders bevorzugt 380 bis 440 °C durchgeführt. Bei der Verwendung von Propan wird das erfindungsgemäße Verfahren bevorzugt zwischen 250 und 350 °C ausgeführt. Bei der Verwendung von Benzol wird das erfindungsgemäße Verfahren bevorzugt zwischen 330 und 450 °C ausgeführt.

Das erfindungsgemäße Verfahren wird vorteilhaft isotherm, mit einer über die Reaktorlänge ansteigender Temperaturführung oder mit einer Kombination aus über die Reaktorlänge ansteigender Temperaturführung und isothermer Fahrweise durchgeführt.

Das erfindungsgemäße Verfahren wird vorteilhaft bei einem Sauerstoffpartialdruck von 0,6 bar bis 50 bar, bevorzugt 2 bar bis 50 bar, besonders bevorzugt 3 bar bis 50 bar, insbesondere 4 bar bis 50 bar durchgeführt.

Der im Vergleich zum Stand der Technik erhöhte Sauerstoffpartialdruck kann vorteilhaft entweder durch die Erhöhung des Gesamtdrucks auf 3 bar bis 50 bar, bevorzugt 5 bar bis 50 bar, insbesondere 10 bar bis 50 bar oder durch eine erhöhte Sauerstoff-Dosierung von 20 Vol.-% bis 98 Vol.-%, bevorzugt 40 Vol.-% bis 98 Vol.-%, insbesondere 60 Vol.-% bis 98 Vol.-% oder durch eine Kombination aus der Erhöhung des Gesamtdrucks und der Sauerstoff-Dosierung erreicht werden.

Beim erfindungsgemäßen Verfahren beträgt das Verhältnis von Sauerstoff zu Kohlenwasserstoff im Eduktstrom vorteilhaft 10 bis 50, bevorzugt 20 bis 50, insbesondere 30 bis 50.

Die n-Butan-Konzentration des der Reaktor-Einheit zugeführten Eingangsstroms beträgt 0,5 bis 10 Vol.-%, bevorzugt 0,8 bis 10 Vol.-%, besonders bevorzugt 1 bis 10 Vol.-% und ganz besonders bevorzugt 2 bis 10 Vol.-%.

Eine hohe n-Butankonzentration von 2 bis 10 Vol.-%, bevorzugt 2,5 bis 10 Vol.-% ist, insbesondere bei hohen Verhältnissen von Sauerstoff zu Butan von vorteilhaft größer 10, bevorzugt größer 20, bevorzugt.

Der n-Butan-Umsatz pro Reaktordurchgang beträgt 40 bis 100 %, bevorzugt 50 bis 95 %, besonders bevorzugt 70 bis 95 % und insbesondere 85 bis 95 % des n-Butans aus dem Eingangsstrom.

Beim erfindungsgemäßen Verfahren stellt man über die Menge des Eingangsstroms in der Reaktor-Einheit eine GHSV (gas hourly space velocity) von bevorzugt 2000 bis 10000 h⁻¹ und besonders bevorzugt 3000 bis 8000 h⁻¹, bezogen auf das auf 0 °C und 0,1013 MPa abs normierte Volumen des zugeführten Eingangsstroms und bezogen auf das Reaktionsvolumen, das mit Katalysator befüllt ist bzw. dessen geometrischen Oberfläche beschichtet ist.

Das erfindungsgemäße Verfahren kann in zwei bevorzugten Verfahrensvarianten, der Variante mit "geradem Durchgang" und der Variante mit "Rückführung" durchgeführt werden. Beim "geraden Durchgang" wird aus dem Reaktoraustrag Maleinsäureanhydrid und gegebenenfalls oxygenierte Kohlenwasserstoff-Nebenprodukte entfernt und das verbleibende Gasgemisch ausgeschleust und gegebenenfalls thermisch verwertet. Bei der "Rückführung" wird aus dem Reaktoraustrag ebenfalls Maleinsäureanhydrid und gegebenenfalls oxygenierte Kohlenwasserstoff-Nebenprodukte entfernt, das verbleibende Gasgemisch, welches nicht-umgesetzten Kohlenwasserstoff enthält, ganz oder teilweise zum Reaktor rückgeführt. Eine weitere Variante der "Rückführung" ist die Entfernung des nicht-umgesetzten Kohlenwasserstoffs und dessen Rückführung zum Reaktor.

Die Umsetzungsprodukte bzw. der Produktstrom können ggf. durch Zugabe von bei den entsprechenden Reaktionsbedingungen inerten Stoffe wie beispielsweise Wasser oder Stickstoff am Ende des Reaktors oder am Reaktorausgang verdünnt werden, so dass ein nicht-explosiver Produktstrom erhalten wird. Ferner kann vorteilhaft ein nicht-explosiver Produktstrom durch eine Druckstufe erreicht werden. Dieser Produktstrom lässt sich dann mit den konventionellen Aufarbeitungseinheiten aufbereiten.

Unter Verwendung von n-Butan wird zur Gewährung einer langen Katalysatorstandzeit und weiteren Erhöhung von Umsatz, Selektivität, Ausbeute, Katalysator-Belastung und Raum/Zeit-Ausbeute dem Gas beim erfindungsgemäßen Verfahren vorteilhaft eine flüchtige Phosphorverbindung zugeführt. Ihre Konzentration beträgt zu Beginn, d.h. am Reaktoreingang 0,2 bis 20 Volumen-ppm der flüchtigen Phosphorverbindungen bezogen auf das Gesamtvolumen des Gases am Reaktoreingang. Bevorzugt ist ein Gehalt 0,5 bis 5 Volumen-ppm. Als flüchtige Phosphorverbindungen sind all jene Phosphorenthaltende Verbindungen zu verstehen, welche in der gewünschten Konzentration unter den Einsatzbedingungen gasförmig vorliegen. Bevorzugt werden als flüchtige Phosphorverbindung Triethylphosphat oder Trimethylphosphat eingesetzt.

Für die Durchführung des erfindungsgemäßen Verfahrens eignen sich allgemein bekannte Mikrokanal-Reaktoren. Im Gegensatz zu konventionellen Reaktionsapparaten, z.B. Rohr(bündel)- oder Wirbelbett-Reaktoren, bieten Mikrokanal-Reaktoren aufgrund der sehr kleinen Abmessungen der Reaktionskanäle (Dimension in mindestens einer Raumrichtung < 3 mm, bevorzugt ca. 1 mm und weniger) eine inhärente Sicherheit, d.h. eine Ausbreitung von Flammen bzw. Explosionen ist nicht möglich (Unterschreiten des minimalen Quenchdurchmessers). Für die Prozessführung bedeutet dies eine größere Freiheit hinsichtlich der Wahl des Verhältnisses Organik / Sauerstoff bzw. Luft, da Explosionsgrenzen innerhalb des Reaktors nicht berücksichtigt bzw. nicht eingehalten werden müssen. Eine Reaktorauslegung auf maximale Explosionsdrücke entfällt. Weiterhin führen kurze Diffusionswege innerhalb der Mikrostrukturen zu stark verbesserten Stoff- und Wärmeübergängen, die um ein Vielfaches über jenen von herkömmlichen Reaktionsapparaten liegen können. Entsprechend entfallen weitgehend Transportlimitierungen, die häufig in konventionellen Rohrbündelreaktoren auftreten. Darüber hinaus ermöglicht das hohe Wärmeabfuhrpotenzial von Mikrokanal-Reaktoren eine präzisere Temperaturkontrolle, so dass z.B. die Ausbildung von Hot Spots unterdrückt und eine Fahrweise mit einem optimal gewählten axialen Temperaturprofil ermöglicht werden kann. Das Durchgehen des Reaktors wird wirkungsvoll unterbunden.

Umfangreiche Beschreibungen zur Gestaltung von Mikrokanal-Reaktoren, welche sich hinsichtlich ihres grundsätzlichen Aufbaus für die Durchführung des erfindungsgemäßen Verfahrens eignen, finden sich beispielsweise in der US 2006/0036106 A1 sowie in der WO 02/18042 A1, worauf hiermit Bezug genommen wird.

Unter Mikrokanal-Reaktoren bzw. Mikroreaktoren, werden allgemein Reaktoren verstanden, deren charakteristischen Abmessungen der Reaktionskanäle, d.h. die Abmessungen in zumindest einer Raumrichtung, z.B. Höhe oder Breite bzw. Durchmesser, im Bereich von einigen Mikrometern bis wenigen Millimetern, bevorzugt < 3 mm liegen.

Auch in großtechnischen Anwendungen bleiben die charakteristischen Abmessungen des Reaktionsraumes erhalten. Die Kapazitätserweiterung erfolgt durch Numbering-up, so dass kosten- und zeitintensives Scale-up entfällt. Die Größe einer Produktionsanlage ist somit flexibel und kann bedarfsgerecht und kostengünstig angepasst werden.

Zur Einbringung der Katalysatoren in den Mikrokanal-Reaktor können alle dem Fachmann bekannten Methoden verwendet werden. Eine umfassende Beschreibung des Standes der Technik dazu findet sich in WO 01/12312 A2 und den darin genannten Referenzen. Der Katalysator kann z. B. als Wandbeschichtung, welche fest mit der Wand des Mikroreaktors verbunden ist (siehe WO 01/12312 A2, Seite 1 und 2 sowie Referenzen darin), vorliegen oder in Form von Splitt oder Formkörpern als Festbettschüttung in den Kanälen des Mikroreaktors eingebracht sein (siehe Tonkovich et al. (Referenz in WO 01/12312 A2, Seite 2)). Ferner kann der Katalysator als Einsatzstück (Insert) beispielsweise in Form einer Metallfolie oder eines Metallgewebes oder Metallnetzes vorliegen, das vorteilhaft mit einer für die katalytischen Eigenschaften geeigneten Oberfläche (z.B. einer Metalloxidoberfläche) versehen ist. Vorteilhaft wird die aktive Komponente auf dieser Oberfläche aufgebracht bzw. fixiert (siehe WO 01/12312 A2, insbesondere Seiten 6 und 7, Kurzbeschreibungen der Abbildungen).

Im Fall einer Festbettschüttung weist der beim erfindungsgemäßen Verfahren einsetzbare Katalysator vorteilhaft Formkörper mit einer im Wesentlichen sphärischen Geometrie auf.

Als Katalysatoren können in Mikrokanal-Reaktoren alle zur Herstellung von Maleinsäureanhydrid allgemein geeigneten Katalysatoren, ggf. mit einem geeigneten Trägermaterial, verwendet werden. Bevorzugt werden solche Katalysatoren eingesetzt, die zur Umsetzung von n-Butan, Propan oder Benzol zu Maleinsäureanhydrid geeignet sind.

Bei der Herstellung von Maleinsäureanhydrid aus n-Butan werden vorteilhaft Vanadium-, Phosphor- und Sauerstoff-enthaltende Katalysatoren mit einem PhosphorNanadium-Atomverhältnis von 0,9 bis 1,5, bevorzugt von 0,9 bis 1,2, insbesondere von 1,0 bis 1,1 eingesetzt. Die mittlere Oxidationsstufe des Vanadiums beträgt vorteilhaft +3,9 bis +4,4 und bevorzugt 4,0 bis 4,3. Die erfindungsgemäßen Katalysatoren besitzen vorteilhaft eine BET-Oberfläche von > 15 m²/g, bevorzugt von > 15 bis 50 m²/g und insbesondere von > 15 bis 40 m²/g. Sie weisen vorteilhaft ein Porenvolumen von > 0,1 ml/g, bevorzugt von 0,15 bis 0,5 ml/g und insbesondere von 0,15 bis 0,4 ml/g. Die Schüttdichte der erfindungsgemäßen Katalysatoren beträgt vorteilhaft 0,5 bis 1,5 kg/l und bevorzugt 0,5 bis 1,0 kg/l.

Die Katalysatoren können die Vanadium, Phosphor und Sauerstoff enthaltende Aktivmasse beispielsweise in reiner, unverdünnter Form als sogenannter "Vollkatalysator" oder verdünnt mit einem bevorzugt oxidischen Trägermaterial als sogenannter "Mischkatalysator" enthalten. Als geeignete Trägermaterialien für die Mischkatalysatoren seien beispielsweise Aluminiumoxid, Siliziumdioxid, Alumosilikate, Zirkondioxid, Titandioxid oder Gemische davon genannt. Bevorzugt sind Vollkatalysatoren.

Die Katalysatoren können ferner weitere Promotoren enthalten. Als Promotoren sind die Elemente der 1. bis 15. Gruppe des Periodensystems sowie deren Verbindungen geeignet. Geeignete Promotoren sind beispielsweise in WO 97/12674 und WO 95/26817 sowie in US 5,137,860, US 5,296,436, US 5,158,923 und US 4,795,818 beschrieben. Bevorzugt werden als weitere Promotoren Verbindungen der Elemente Molybdän, Eisen, Zink, Hafnium, Zirkon, Titan, Chrom, Mangan, Nickel, Kupfer, Bor, Silizium, Zinn, Niob, Kobalt, Lithium, Antimon und Wismut, insbesondere Molybdän, Eisen, Zink, Wismut. Der Gehalt an Promotoren beträgt in Summe im fertigen Katalysator im allgemeinen nicht mehr als etwa 5 Gew.-%, jeweils als Oxid gerechnet.

Die Katalysatoren können auch sogenannte Hilfsmittel, wie etwa Tablettierhilfsmittel oder Porenbildner enthalten.

Ferner können als katalytisch aktive Masse auch dem Fachmann bekannte Heteropolysäuren verwendet werden. Bei n-Butan-Umsätzen von 15 % werden üblicherweise Selektivitäten zu Maleinsäureanhydrid von 90 % bzw. bei Umsätzen von 62 % werden üblicherweise Selektivitäten von 46 % zu Maleinsäureanhydrid erzielt (Davis et al., Angew. Chem. (2002) 114, 886-888; Holles et al., J. Catal. (2003) 218, 42-66).

Die genannten Katalysatoren können nach allen dem Fachmann bekannten Verfahren hergestellt werden. Die erfindungsgemäßen Katalysatoren können beispielsweise wie in den Patentschriften US 5,275,996 und US 5,641,722 oder der Offenlegungsschrift WO 97/12674 beschrieben hergestellt werden. Die Formgebung erfolgt bevorzugt durch Tablettierung.

Die Katalysatorherstellung wird im Stand der Technik im Allgemeinen als ein mehrstufiger Prozess beschrieben, bei dem man zunächst einen sogenannten Katalysatorprecursor herstellt und diesen anschließend durch Kalzinierung in die aktive Form überführt. Die beim erfindungsgemäßen Verfahren einsetzbaren Katalysatorprecursor können beispielsweise wie in den Schriften US 5,275,996, US 5,641,722, WO 97/12674, WO 01 /68626, WO 01 /68245, WO 02/22257, WO 02/34387, DE 102 11 449 A1, DE 102 11 445 A1, DE 102 11 447 A1, DE 102 11 446 A1 und DE 102 35 355 A1 beschrieben hergestellt werden.

Die Präformierung kann im erfindungsgemäßen Verfahren, im Gegensatz zum Stand der Technik, direkt im Mikrokanal-Reaktor stattfinden.

Bei der Herstellung von Maleinsäureanhydrid aus Propan werden vorteilhaft Katalysatoren auf der Basis von Vanadium- und Molybdän-Mischoxiden eingesetzt (Tang et al., Appl. Catal. A 287 2005 197). Das V / Mo-Verhältnis variiert vorteilhaft zwischen 1/9 bis 3/7, bevorzugt 1/4 bis 3/7. Die Aktivmasse kann vorteilhaft neben den beiden Hauptkomponenten Vanadium und Molybdän noch Dotierungskomponenten zur Steigerung der Aktivität bzw. Selektivität des Katalysators. Besonders bevorzugt werden als Dotierungskomponenten Ag, Cu und Zn verwendet. Das Verfahren zur Herstellung des Katalysators erfolgt nach dem Fachmann bekannten Methoden, die beispielsweise in (Tang et al., Appl. Catal. A 287 2005 197) beschrieben sind.

Bei der Herstellung von Maleinsäureanhydrid aus Benzol werden vorteilhaft Schalenkatalysatoren mit geträgerter Aktivmassen auf der Basis von Molybdän- und Vanadium-Mischoxiden eingesetzt. Das Mo/V-Verhältnis variiert vorteilhaft zwischen 1/2,5 bis 1/5. Der Aktivmassenanteil des Schalenkatalysators liegt vorteilhaft bei 10 bis 20 Gew.-%. Die Aktivmasse kann vorteilhaft neben den beiden Hauptkomponenten Molybdän und Vanadium noch Dotierungskomponenten zur Steigerung der Aktivität bzw. Selektivität des Katalysators enthalten. Als Dotierungskomponenten werden vorteilhaft Ag, Na (Bielanski et al., Bull. Acad. Pol. Sci., Ser. Sci. Chim. (1976) 24(5), 415-23), Seltene Erden wie beispielsweise Tb, Dy, Gd oder Er (Khiteeva et al., Zh. Fiz. Kihm. (1981) 55(8), 2121-2), Cr, Co (Bielanski et al., Bull. Acad. Pol. Sci. Ser. Sci. Chim. (1976) 24(6), 485-92, Zn, Cu (Ionita et al., Rev. Chim. (Bucharest) (1968) 19(2), 105-7) verwendet. Auch andere Promotoren, die in der Oxidation von o-Xylol zu Phthalsäureanhydrid verwendet werden, wie z.B. Cs und P, können vorteilhaft eingesetzt werden. Das Verfahren zur Herstellung des Katalysators erfolgt nach dem Fachmann bekannten Methoden, die beispielsweise in Ullmann's Encyclopedia of Industrial Chemistry, 6th edition, 2000 electronic release, Kapitel 2.2.1 beschrieben sind.

Das erfindungsgemäße Verfahren weist eine im Vergleich zum Stand der Technik um 10 % verbesserte Ausbeute auf. Ferner kann der separate Verfahrensschritt der Präformierung eingespart werden, da die Präformierung direkt im Mikrokanal-Reaktor durchgeführt werden kann.

## Patentansprüche

1. Verfahren zur Herstellung von Maleinsäureanhydrid durch heterogenkatalytische Gasphasenoxidation eines Kohlenwasserstoffs, **dadurch gekennzeichnet, dass** man einen Kohlenwasserstoff enthaltenden Stoffstrom und einen Sauerstoff oder eine Sauerstoffquelle enthaltenden Stoffstrom einem Mikrokanal-Reaktor zuleitet und in dem den Katalysator enthaltenden Mikrokanal-Reaktor die Umsetzung zu Maleinsäureanhydrid im Explosionsbereich erfolgt.

2. Verfahren nach Anspruch 1, **dadurch gekennzeichnet, dass** die Umsetzung zu Maleinsäureanhydrid bei einem Sauerstoffpartialdruck von 0,6 bis 50 bar erfolgt.

3. Verfahren nach Anspruch 1 oder 2, **dadurch gekennzeichnet, dass** die Umsetzung zu Maleinsäureanhydrid bei einem Gesamtdruck von 3 bis 50 bar erfolgt.

4. Verfahren nach den Ansprüchen 1 bis 3, **dadurch gekennzeichnet, dass** die Umsetzung zu Maleinsäureanhydrid bei einer Sauerstoff-Dosierung von 20 bis 98 Vol.-% erfolgt.

5. Verfahren nach den Ansprüchen 1 bis 4, **dadurch gekennzeichnet, dass** die Umsetzung zu Maleinsäureanhydrid bei einem Verhältnis von Sauerstoff zu Kohlenwasserstoff von 10 bis 50 erfolgt.

6. Verfahren nach den Ansprüchen 1 bis 5, **dadurch gekennzeichnet, dass** als Kohlenwasserstoff n-Butan, Propan oder Benzol eingesetzt wird.

7. Verfahren nach den Ansprüchen 1 bis 5, **dadurch gekennzeichnet, dass** als Kohlenwasserstoff n-Butan und als Katalysator ein Vanadium-, Phosphor- und Sauerstoff-enthaltenden Katalysator mit einem Phosphor/Vanadium-Atomverhältnis von 0,9 bis 1,5 verwendet wird.

8. Verfahren nach den Ansprüchen 1 bis 7, **dadurch gekennzeichnet, dass** der Katalysatorprecursor im Mikrokanal-Reaktor präformiert wird.
